# EUROPEAN PATENT SPECIFICATION

(11) **EP 0 693 933 B1**
(45) Date of publication and mention of the grant of the patent: **26.06.2002**
(21) Application number: 94914804.3
(22) Date of filing: 15.04.1994
(51) Int. Cl.: A61K 38/08, A61K 38/17, A61K 38/28, A61K 39/39

(54) **METHODS AND COMPOSITIONS FOR DELAYING OR PREVENTING THE ONSET OF AUTOIMMUNE DISEASE**
VERFAHREN UND ZUBEREITUNGEN ZUR VERZOEGERUNG ODER VORBEUGUNG VON AUTOIMMUN KRANKHEITEN
PROCEDES ET COMPOSTIONS PERMETTANT DE RETARDER OU DE PREVENIR L'APPARITION D'UNE MALADIE AUTO-IMMUNE

(30) Priority: 16.04.1993 US 48979
(43) Date of publication of application: 31.01.1996
(73) Proprietor: RESEARCH CORPORATION TECHNOLOGIES, INC., Tucson, Arizona 85711-3335 (US)
(72) Inventor: MACLAREN, Noel K., University of Florida, Gainesville, FL 32610 (US); MUIR, Andrew, Gainesville, FL 32605 (US)
(74) Representative: Fisher, Adrian John
(86) International application number: PCT/US94/04179
(87) International publication number: WO 94/23737

(56) References cited:
- EP-A- 0 055 885
- US-A- 4 789 660
- NATURE. vol. 366 , 4 November 1993 , LONDON GB pages 69 - 72 DANIEL L. KAUFMAN ET AL. 'SPONTANEOUS LOSS OF T-CELL TOLERANCE TO GLUTAMIC ACID DECARBOXYLASE IN MURINE INSULIN-DEPENDENT DIABETES' cited in the application
- NATURE. vol. 366 , 4 November 1993 , LONDON GB pages 72 - 75 ROLAND TISCH ET AL. 'IMMUNE RESPONSE TO GLUTAMIC ACID DECARBOXYLASE CORRELATES WITH INSULITIS IN NON-OBESE DIABETIC MICE' cited in the application
- DATABASE WPI Section Ch, Week 9201, Derwent Publications Ltd., London, GB; Class B04, AN 92-005453 & SU,A,1 624 511 (DON MED INST) 30 January 1991

## Description

### Background of the Invention

Autoimmune diseases are characterized by an abnormal immune response (involving either immune system cells or antibodies) directed against normal autologous (self) tissues. Autoimmune diseases afflict huge numbers of individuals throughout the world.

A normal immune system has the capacity to identify and destroy a large variety of foreign invader organisms such as bacteria and viruses. Remarkably, a normal immune system can readily distinguish foreign substances from self, and thereby is able to react vigorously against potentially pathogenic entities from the environment without harming the host's own cells.

The immune system's non-reactivity to self is termed immunological tolerance. In pathological situations, immunological tolerance to a wide variety of self substances is broken, resulting in an autoimmune response. If of an appropriate nature and of sufficient severity and duration, the anti-self response will result in an autoimmune disease. In certain autoimmune diseases, specific elements of the immune system predominate in mediating the pathogenic process, while in other autoimmune disease, all of the components of the immune system cooperate to produce disease. Antibodies are considered to play the major causal roles in diseases such as systemic lupus erythematosus, myasthenia gravis and Graves' disease, while cellular immune mechanisms are believed to be those primarily involved in multiple sclerosis (MS) and insulin dependent diabetes (IDD).

Whereas susceptibility to autoimmune diseases may be inherited through the defective actions of multiple genes, indirect evidence suggests that an interaction with a foreign substance from the environment may also be necessary to induce the pathogenic process that results in disease. One explanation for this is that immunization with the foreign inductive chemical induces a cross-reactive response to self through molecular mimicry or chemical similarity. However, once the autoimmune process has been initiated, other secondary immunizing events involving other self antigens typically occur through the release of intracellular constituents in forms not normally encountered by the immune system. Targeted organs thus become damaged through the combination of all of these events, which leads to the appearance of a clinically recognized disorder only when the disease process has progressed to ablate large numbers of tissue cells so targeted.

A number of strategies have been used or proposed to suppress autoimmune diseases, most notably drugs, such as cyclophosphamide, cyclosporin A, methotrexate, and Imuran (azathioprine). Steroid compounds, such as prednisone and methylprednisolone, are also employed in many instances. These drugs have limited long term efficacy against both cell- and antibody-mediated autoimmune diseases. Use of such drugs is limited by virtue of their toxic side effects which include "global" immunosuppression. Prolonged treatment with these drugs inhibits the normal protective immune response to pathogenic microorganisms, thereby increasing the risk of infections. A further drawback is that immune-mediated elimination of aberrant cells is impaired and there is, thus, an increased risk that malignancies will develop in patients receiving prolonged global immunosuppression.

The self substances, or autoantigens, which are the targets of autoimmune responses are most often protein products unique to the targeted cells (e.g. hormones such as insulin in IDD); particular enzymes unique to the specialized function of targeted cells (e.g., glutamic acid decarboxylase or GAD in IDD, or 21 hydroxylase in Addison's disease); specialized cell-specific receptor molecules (e.g. the thyroid stimulating hormone (TSH) receptor in Graves' disease or acetylcholine receptors in the neuromuscular junctions in myasthenia gravis); and/or structural constituents of the targeted cells or tissues (e.g., beta cell sialo-glycoconjugate in IDD). Prior to the current invention, immunization with autoantigens has been used as a means to induce autoimmune disease in experimental animals. For example, the administration of myelin basic protein (MBP) has been used as a means to induce experimental allergic encephalomyelitis (EAE, a model for MS) in mice.

The methods and compositions of the subject invention relate to the prevention and/or treatment of IDD. A detailed background of diabetes is provided below.

Diabetes mellitus comprises a group of diseases that result in elevations of blood glucose levels because of relative to absolute deficiencies in the pancreatic hormone insulin. Insulin is secreted into the blood when food is ingested and primarily directs absorbed nutrients into body stores. Diabetes is a major public health problem affecting at least 5 million and as many as 10 million Americans. The prevalence of the most severe form of IDD is 1 in 300 in the United States. In 1991, the direct health care costs attributable to diabetes care in the United States exceeded $20 billion, with as much as twice this figure in additional indirect costs, such as loss of productivity (Bransome and Edwin (1992) Diabetes *Care* 15: 1-5).

Chronic elevation of blood glucose levels is the most obvious metabolic effect in diabetes and is associated with progressive damage to blood vessels. This leads to heart attacks, strokes, blindness, peripheral nerve dysfunction, and kidney failure. The frequency and severity of diabetes-related complications are greatest in the insulin dependent form of the disease, in which an immunological destruction of the insulin-secreting pancreatic beta cells occurs. The high rate of irreversible complications in IDD occurs despite the availability of insulin replacement through injections given 1-4 times daily.

Insulin and other pancreatic hormones are well known and characterized. See, for example, Steiner et al. (1989) "Chemistry and Biosynthesis of Pancreatic Protein Hormones," in *Endocrinology;* DeGroot et al., Eds., W.B. Saunders Company, p. 1263-1289. As described in Steiner et al., the amino acid sequence of insulin is highly conserved across a number of species, including human, monkey, porcine, and bovine. Although insulin is well known for its association with diabetes, there are a number of other proteins, including glucagon and glutamic acid decarboxylase (GAD), which are also associated with the pancreas or diabetes.

Through the research efforts of applicants and others, IDD has proved itself to be predictable both in unaffected relatives of patients with IDD, as well as in persons from the general population. A predisposition to develop clinical diabetes can be determined through several different tests. For example, genetic susceptibility to diabetes has become increasingly definable through the use of molecular biological means, usually from DNA samples obtained from peripheral blood. One major gene involved in the inherited susceptibility to IDD is that located at the HLA-DQ locus. It is currently possible to identify risks varying from essentially none to those as high as 70 fold above those without the genotype. In families a genetic risk as high as 1 in 4 can be estimated for unaffected siblings just through identification of HLA haplotypes shared with the affected proband.

Persons who have just developed IDD or are in process of developing IDD have a number of disease-specific autoantibodies in their blood. Such autoantibodies include those to islet cell antigens (ICA), to beta cell specific proteins of 64 kDa, which are now believed to be the lower molecular isoform of glutamic acid decarboxylase (GAD₆₅), to native insulin and proinsulin, and to a number of more minor determinants such as carboxypeptidase-H and heat shock proteins belonging to the hsp-60 family.

Insulin autoantibodies (IAA) are observed in untreated, newly diagnosed IDD patients (Palmer et al. (1983) *Science* 222: 1337-1339) as well as in apparently unaffected relatives of diabetic probands. Whereas autoimmunity to insulin could directly cause β-cell damage, interfere with the action of endogenous insulin, or have both effects, some investigators suggested that IAA reflect the rate of islet cell destruction and thus act merely as reporters of aggressive islet directed autoimmunity (Ziegler et al. (1989) *Diabetes* 38: 1320-1325; Vardi (1988) *Diabetes Care* 11: 736-739).

The non-obese diabetic (NOD) mouse is a useful animal model for human IDD. Analysis of the NOD mouse provides important insights into the sequence of pathogenic events, and leads to an understanding of the nature of the target islet cell autoantigens involved in the autoimmunological process. Previous studies from the inventors' laboratory have demonstrated that an extended prophylactic course of daily, subcutaneous injections of porcine insulin protected NOD mice from both hyperglycemia and islet infiltration by mononuclear leukocytes (insulitis) (Atkinson et al (1990) *Diabetes* 39: 933-937). Such treatment may relieve the pancreatic β-cells of metabolic demands and thus induce a state of "β-cell rest". This quiescent state may be associated with diminished expression of many islet factors, including those that may serve as potential autoantigens at the cell surface (Aaen et al (1990) *Diabetes* 39: 697-701; Kämpe et al (1989) *Diabetes* 38: 1326-1328). Non-specific immunostimulation caused by cytokine (Jacob et al (1990) *Proc. Natl. Acad. Sci. USA* 87: 968-972) or adjuvant (Sadelain et al. (1990) *Diabetes* 39: 583-589) treatments, or environmental microbes have been implicated in other protocols of IDD prevention (Like et al. (1990) *Diabetes* 40: 259-262). An enhanced understanding of the pathogenic role of insulin autoimmunity in IDD is clearly required.

There have been reports of efforts to induce antigen-specific immunoregulation to ameliorate autoimmune diseases (Steinman, L. (1990) *Mol. Biol. Med.* 7: 333-339). For example, various methods have been employed to induce antigen-specific suppression of experimental allergic encephalomyelitis (EAE) (PCT application WO 91/15225). Recently, several novel immunological approaches have been explored to autoimmune diseases such as EAE in mice and rats and lupus nephritis in MRL/lpr mice. Many have been directed toward blocking the function of the effector CD4⁺ T cell which has been shown to exhibit V_{β} isotype restriction in EAE. These approaches have included the use of anti-TCR antibodies (Archa-Orbea et al., supra), synthetic TCR peptides (Offner et al. (1991) *Science* 251: 430-432) and superantigen treatment (Kim et al. (1991) *J. Exp. Med.* 174: 1431-1437). The tolerogenic effects of intragastric antigens' administration have also been described (Silverman et al. (1983) *J. Immunol.* 131: 2651-2661; Peng et al. (1990) *Clin. Exp. Immunol.* 81: 510-515; Michael (1989) *Immune Invest.* 18: 1049-1054; Kitamura et al. (1987) *J. Immunol.* 139: 3251-3259; Michalek et al. (1982) *J. Immunol.* 128: 1992-1998; Mowat et al. (1986) *Immunol.* 58: 677-683; PCT applications WO 91/12816, WO 91/01333; WO 92/06704). Nagler-Anderson et al. (1986) *Proc. Natl. Acad. Sci. USA* 83: 7443-7446 describe the suppression of arthritis by oral administration of soluble type II collagen in a collagen-induced arthritis mouse model. Deficiencies in this ability have been reported in several experimental mouse models of autoimmune diseases (Gesualdo et al. (1990) *J. Immunol.* 145: 3684-3691; Miller et al. (1984) *Clin. Immunol. Immunopathol.* 31: 231-240).

Zhang et al. observed a beneficial response to oral insulin (Zhang et al. (1991) Proc. *Natl. Acad. Sci. USA* 88: 10252-10256) and further demonstrated in co-transfer studies that splenocytes from insulin-fed NOD mice prevented the adoptive transfer of diabetes by splenocytes from untreated diabetic mice to irradiated recipients.

The current invention provides the means for the first time to prevent the disease process from becoming established in the genetically predisposed, or otherwise in progressing once initiated as identifiable by autoantibodies, through a novel treatment that is akin to vaccination with self proteins or peptide epitopes thereof important in the pathogenic process underlying IDD.

### Brief Summary of the Invention

The subject invention concerns unique compositions and the use of an adjuvant and an antigen in the preparation of a composition useful in the suppression of immune-mediated destruction of pancreatic tissue in a manonal suffering from or susceptible to insulin dependent diabetes, said antigen being the B chain of insulin or a fragment thereof, and said composition being adapted for administration by parenteral injection for the prevention, delay in the onset of, or early amelioration of insulin dependent, or Type 1, diabetes (IDD). This procedure is particularly advantageous when used to vaccinate persons found to be at genetic risk for, or in the process of, developing IDD. The vaccination procedure disclosed herein effectively blocks or immunologically overrides the autoimmune reaction that causes destruction of the insulin-secreting cells of the pancreas.

A primary principle of the vaccination methods of the subject invention is that the antigen used to immunize is one that is targeted by the immunological disease or else is one uniquely present in the particular body tissues that become damaged by the pathological immune response. The antigen used to vaccinate can be the insulin B chain, an insulin B chain peptide, GAD₆₅, or a GAD₆₅ peptide administered parenterally by injection.

As specifically described herein, an IDD-resistant state in NOD mice is induced by administering insulin B chain subcutaneously with an appropriate adjuvant. Incomplete Freund's adjuvant (IFA) is the adjuvant specifically exemplified herein; other suitable adjuvants can be substituted according to known principles. The incidence and severity of insulitis were little affected, or actually increased, by this procedure. Antibodies and *in vitro* proliferative responses to insulin were stimulated by immunization with insulin in IFA. Despite these immunological responses which have previously been associated with the pathogenesis or disease progression of diabetes, strong protection for diabetes was achieved.

Subcutaneous immunization with the insulin B chain but not the insulin A chain, reproduced the protective effect of insulin, suggesting the existence of immunodominant epitope on the B chain. Preferably, the insulin B chain is combined with a suitable adjuvant, for example, incomplete Freund's adjuvant. Thus, in a preferred embodiment, the vaccination strategy as applied to IDD is particularly advantageous because metabolically inactive insulin fragments can be used. GAD₆₅ or fragments thereof in the presence of a suitable adjuvant parenterally administered can also be used in place of, or in addition to, insulin B chain or peptide therefrom.

The protective effect achieved by the methods of the subject invention is apparently cell-mediated, since protection from IDD was induced by the infusion of splenocytes from acutely diabetic mice donors transferred to irradiated NOD mice recipients by the co-infusion of splenocytes harvested from B chain treated mice. Preferably, the combination of antigen and adjuvant administered parenterally results in the suppression of immune cell-mediated destruction of the target tissue in IDD.

The immunization composition of the subject invention obviates the need for daily insulin therapy, the need for repeated blood glucose monitoring, and the restrictions of diet and exercise imposed upon all patients with IDD receiving insulin replacement therapy. Further, and most importantly, the complications of IDD, which are directly attributable to the duration and severity of overt diabetes and hyperglycemia, will be inhibited. Those who would be likely to benefit most from insulin vaccination therapy would be those with a high genetic risk profile, such as a five-fold or greater risk as determined by HLA and/or other genotyping studies, as well as those found to have a high predictive profile as determined through specific autoantibody analyses, e.g., GAD₆₅ autoantibodies.

### Brief Description of the Drawings

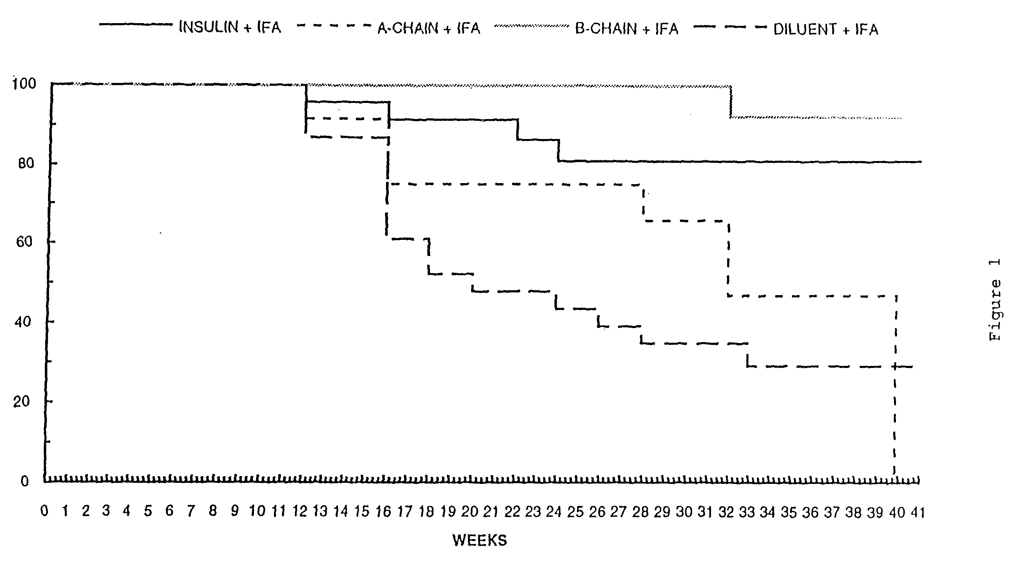

Figure 1 is a survival curve for NOD mice vaccinated at 4 and 8 weeks of age as described herein with insulin + IFA(-), insulin A chain + IFA (---), insulin B chain + IFA (...) and diluent + IFA (_ _), as percent IDD-free vs. age in weeks.

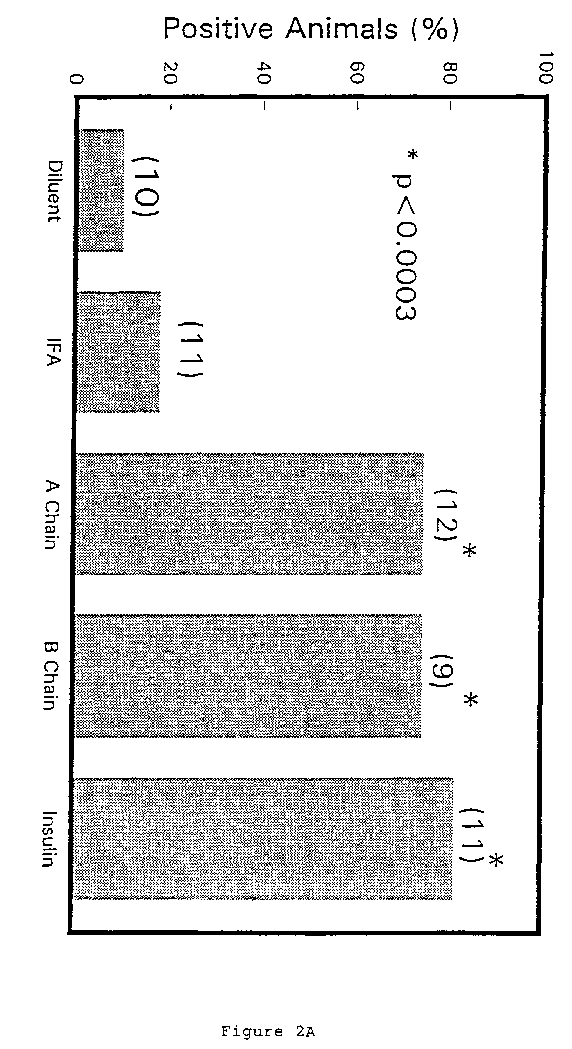

Figure 2A illustrates the percent of NOD mice positive for insulin-specific antibodies after vaccination with diluent alone, IFA alone, insulin A chain + IFA, insulin B chain + IFA, or insulin + IFA. Numbers of animals per treatment are given in parentheses above the bars.

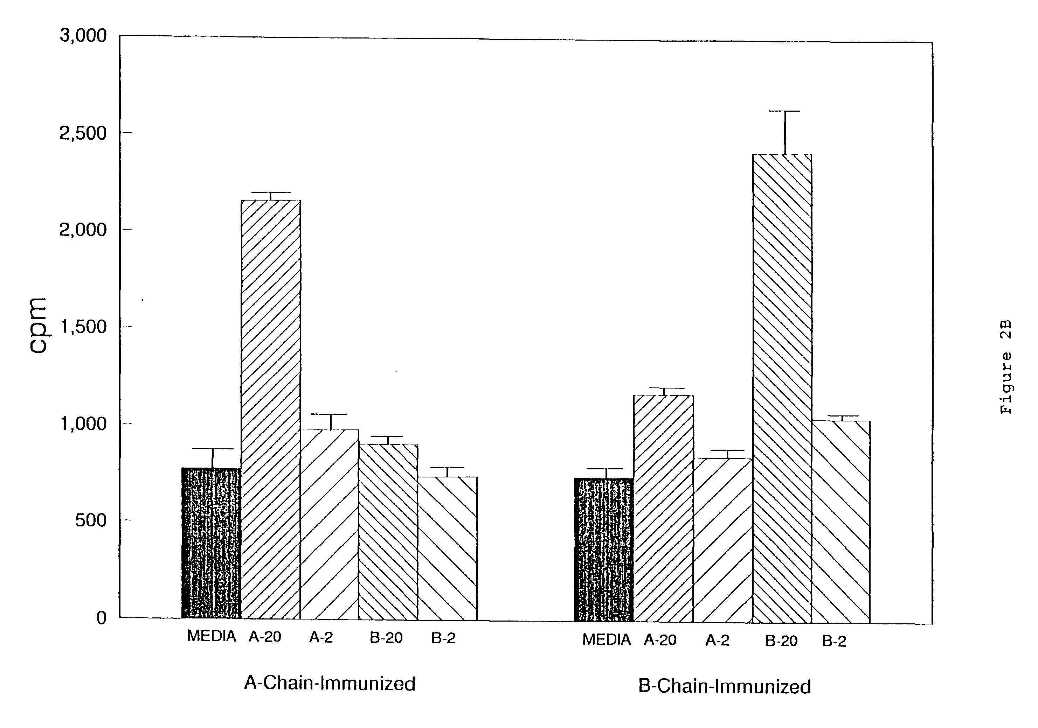

Figure 2B illustrates a representative experiment for splenocyte proliferative responses to insulin A or B chain challenge, as measured by radioactive thymidine incorporation, after immunization of the splenocyte source animals with insulin A chain or B chain, each plus IFA. The levels of thymidine incorporation are shown for each splenocyte population after challenge with medium only, 20 µg/ml A chain insulin (A-20), 2 µg/ml A chain insulin (A-2), 20 µg/ml insulin B chain (B-20) and 2 µg/ml B chain insulin (B-2). The floating bars represent the average value plus one standard deviation.

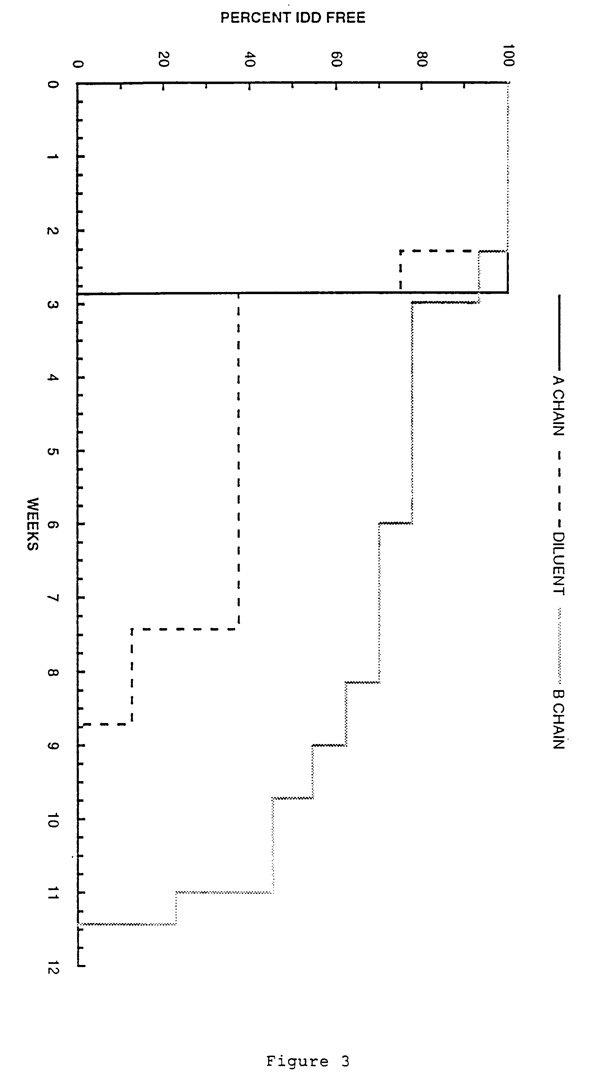

Figure 3 illustrates the percentage of IDD-free (surviving) animals following adoptive co-transfer experiments after prior immunization of the splenocyte donor animals with insulin A chain, diluent or B chain, each with IFA. Protection afforded by B-immunized splenocytes appears transient. The "immune" splenocytes were mixed with IDD splenocytes before introduction into the animals in which survival was measured.

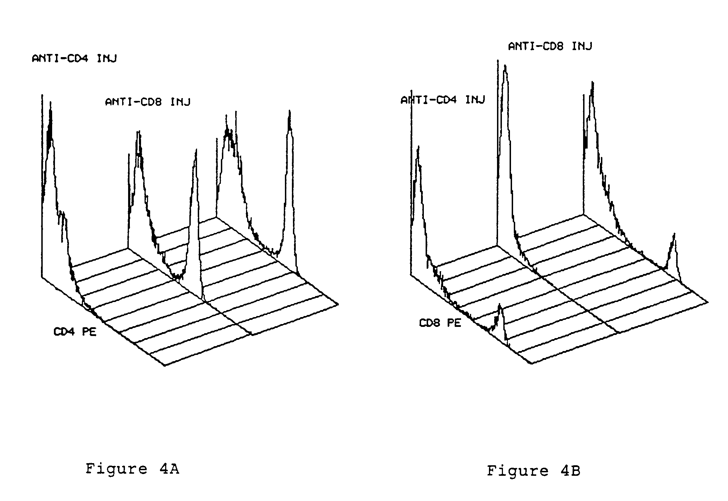

Figure 4A and Figure 4B illustrate the depletion of CD4+ and CD8+ spleen lymphocytes in animals injected with anti-CD4-specific monoclonal antibody or anti-CD8-specific monoclonal antibody. CD4+ and CD8+ lymphocytes are measured by Fluorescence Activated Flow Cytometry. In both Figures 4A and 4B, the leftmost curve is for splenocytes from animals treated with anti-CD4 monoclonal antibody, middle are splenocytes from anti-CD8 treated animals and the rightmost are splenocytes from control animals. In each of Figures 4A and 4B, the vertical axis is number of cells and the axis coming toward the viewer is fluorescence intensity (increasing toward the viewer). The flow cytometry in Figure 4A measured CD4+ splenocytes with the use of anti-CD4-phycoerythrin conjugate and Figure 4B measures CD8+ T cells with the use of anti-CD8-phycoerythrin conjugate. The results show that the target cell populations were effectively removed by the antibody treatments.

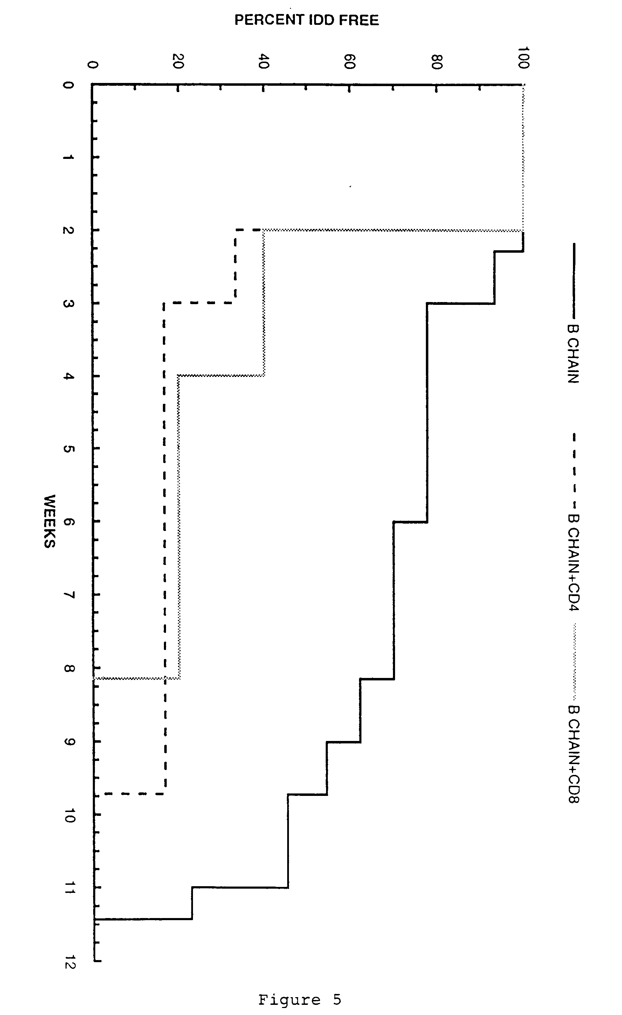

Figure 5 illustrates survival (as percent IDD-free) of NOD mice after adoptive co-transfer experiments and the effect of CD4+ or CD8+ T cell ablation before transfer. All transfers included splenocytes from NOD mice with IDD plus splenocytes from insulin B chain + IFA-immunized mice. Those B chain-immunized mice were divided into three groups: control (solid line), anti-CD8 monoclonal antibody-treated (dotted line) and anti-CD4 antibody-treated (dashed line) before donation. The results demonstrate that both CD4+ and CD8+ lymphocytes are required for the active protective response to insulin B chain plus IFA vaccination, which protective effect lasts about 8 weeks.

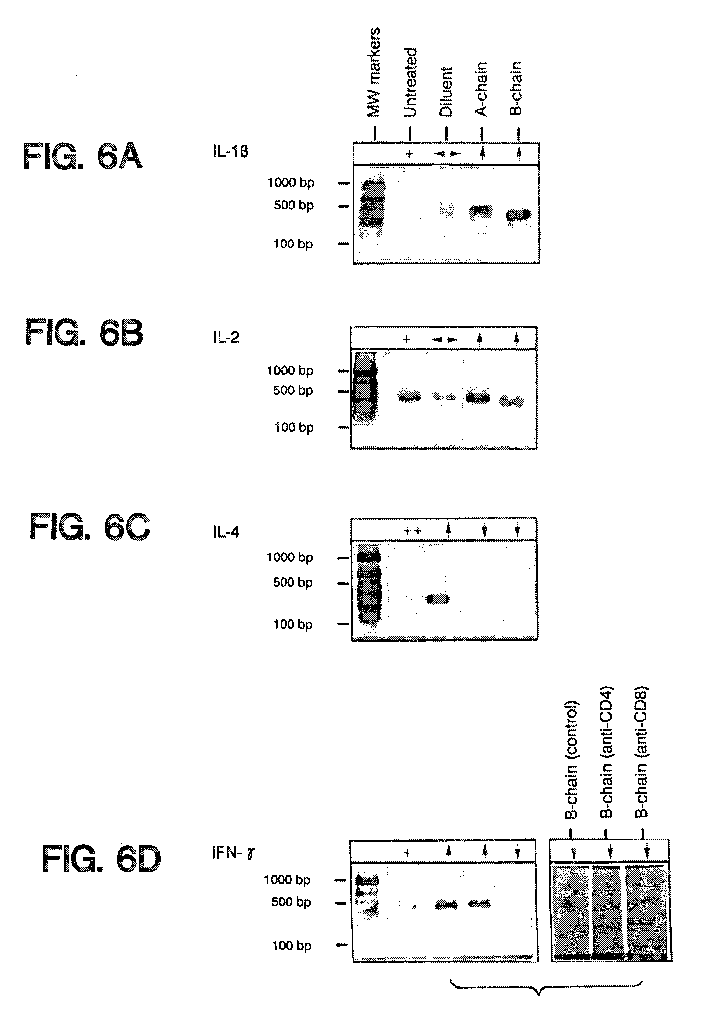

Figure 6A-6D are blots of RT-PCR amplimers visualized with dioxigenin-labeled probes specific for interleukin-1β coding sequences (Fig. 6A), interleukin-2 coding sequences (Fig. 6B), interleukin-4 coding sequences (Fig. 6C), and interferon-γ coding sequences (Fig. 6D). The arrows at the top of each panel indicate the increase (↑), decrease (↓) or insignificant effect 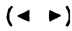 on apparent mRNA abundance in response to the particular vaccination treatment or vaccination/CD4 or CD8 ablation treatment noted for each lane.

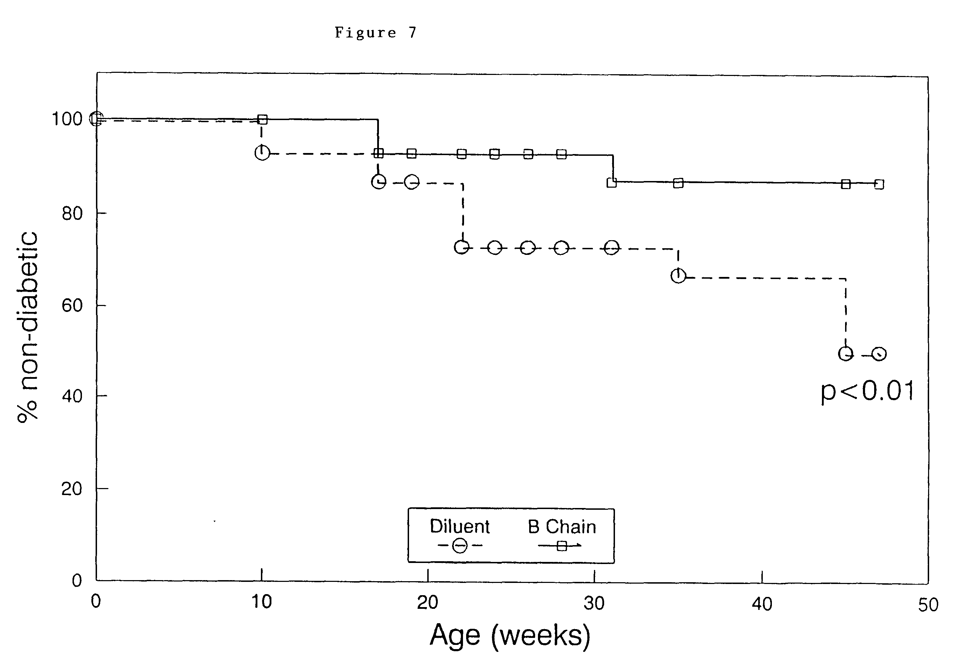

Figure 7 illustrates the percent of NOD mice remaining non-diabetic after vaccination at 10 and 14 weeks with IFA+ diluent (dashed line) or IFA+ insulin B chain (solid line).

### Brief Description of the Sequences

**SEQ ID NO: 1** is an 11-amino acid peptide within the B chain of porcine insulin.

**SEQ ID NO: 2** is a 9-amino acid peptide from the N-terminus of the B chain of porcine insulin.

**SEQ ID NO: 3** is the amino acid sequence for bovine and human insulin B chain.

**SEQ ID NO: 4** is the amino acid sequence for porcine insulin B chain.

### Detailed Disclosure of the Invention

The subject invention concerns a new, clinically important, immunomodulating therapy that selectively inhibits destructive immune responses without inducing generalized immunosuppression replete with its inherent side effects. The treatment procedures of the subject invention represent an important advance in the care of patients suffering from, or predisposed to, autoimmune disorders. These disorders are a disparate collection of human diseases that have in common the failure of the immune system to recognize a tissue, organ or cellular component of the body as self. As the result of these pathological processes, self cells targeted for attack by the immune system become dysfunctional, destroyed with permanent loss of normal biological activities, or occasionally are stimulated to overfunction.

Specifically disclosed herein are prophylactic procedures for the prevention or early amelioration of insulin-dependent diabetes (IDD).

A specific embodiment of the subject invention is a novel vaccination regimen for the prevention or delay in the onset of IDD. The vaccination procedure involves infrequent subcutaneous injections of insulin B chain, or fragments thereof, in a composition which further comprises an adjuvant, for example, incomplete Freund's adjuvant (IFA). Immunization with insulin B chain, but not A chain insulin fragments, resulted in reduced IDD incidence in NOD mice. Splenocytes from mice immunized with insulin B chain conferred the protective effect on irradiated NOD recipients in adoptive co-transfer studies.

As described herein, NOD mice immunized bimonthly with insulin suspended in IFA showed a profound reduction in the frequency of diabetes to under 13% (Figure 1). This effect was not found following injections of mice with adjuvant alone (IDD rate of 52%) or of mice given commercial insulin diluent alone (IDD rate of 70%). Similar immunizations with other IDD irrelevant antigens, including bovine serum albumin or its specific ABBOS peptide implicated by others (Karjalainen et al. (1992) *New Engl. J. Med.* 327:302-307) as an important inducer of human IDD, had no protective effects. However, Atkinson et al. (1993) *New England J. Med.* 329: 1853-1858 reported that immune responsiveness to BSA did not appear to be a significant factor in IDD. Surprisingly, the severity of the insulitis lesion was not significantly reduced by immunization with insulin. This demonstrates that the procedure of the subject invention initiates an active, favorable immune response which overrides the predisposition to develop IDD. This vaccination therapy is in distinct contrast to previous efforts to prevent IDD by dampening the immune response.

The protective effect from insulin immunization is specific to unique portions of the insulin molecule. Specifically, immunizing treatments with the A chain of porcine insulin had no protective effects (IDD rate 53%) while all of the protection was localized to the B chain (IDD rate 16%). Immunizations with both insulin A and B chains resulted in high insulin-binding activity in serum; however, diabetes was prevented only by insulin B chain immunization. In additional studies, the major epitope involved in the protective effect was mapped to an 11-amino acid peptide (gly-ser-his-leu-val-glu-ala-leu-tyr-leu-val) (SEQ ID NO.1) within the B chain. The protective effect of a hormonally inactive, specific epitope on the insulin molecule suggests that beta cell rest is not a factor and that active immunization of lymphocytes is involved. An addition B chain peptide found to have a protective effect is phe-val-asn-gln-his-leu-cys-gly-ser (SEQ ID NO.2). These two peptides overlap within the insulin B chain. Thus, there appears to be a dominant epitope located on the B chain that induces a protective, antigen-specific, regulatory T cell and idiotype-antiidiotype network. Alternatively, the cellular and humoral immune responses may be dichotomous events.

It would be readily apparent to those skilled in the art with the benefit of the subject disclosure that variants of the peptides and proteins disclosed herein could be used according to the subject invention. For example, insulin protein or peptide sequences from other animals could be used. Also, inconsequential amino acid substitutions could be made which did not affect the ability of the protein or peptide to produce the advantageous protective immune response described herein.

The outstanding protective effect of the subcutaneous insulin and insulin B chain vaccinations of the subject invention was associated with very high circulating levels of antigen-specific antibodies. Such antibodies may be distinct from IAA because of epitope specificity, subclass, or isotope differences, and they may therefore also have different biological functions. Thus, these antibodies may competitively antagonize any detrimental effects of IAA. They may also stimulate antiidiotypes that bind the IAA and block their potentially harmful effects.

In a further study, splenic lymphocytes from mice immunized with the B chain of insulin were able to protect irradiated recipient mice co-transferred with spleen cells which otherwise conveyed accelerated diabetes. This strongly suggested that a cell-mediated regulatory action is occurring. Thus, the apparent paradoxical inhibition of autoimmunity by immunization is likely to occur through the stimulation of lymphocyte networks within which immunoregulatory functions predominate and thus actively interfere with the actions of disease-producing clones. Such "suppressive" actions may arise from, for example: (1) release of inhibitory cytokines (e.g., interleukin-10 [IL-10], transforming growth factor-β [TGF-β]); (2) competition for antigen binding between non-destructive and destructive cells recognizing the same antigen; (3) stimulation of "anti-idiotypic" T lymphocytes that bind the TCR of destructive T lymphocytes; and (4) activation of "suppressor-inducer"lymphocytes.

Insulin is only one of many β-cell autoantigens associated with diabetes in NOD mice, but autoimmune responses against the hormone have not been considered necessary for the induction of diabetes. (Atkinson et al. (1993) *J. Clin. Invest.* 92: 1608-1616). The near complete protection from disease after therapeutic immunization with B-chain insulin in IFA is therefore consistent with the concept of "bystander immunosuppression." This term describes antigen specific immunoregulatory cell release of cytokines (including TGF-β) whose paracrine actions down-regulate other autoreactive immune cells in the islet inflammatory lesion, irrespective of their cognizant antigens. (Miller et al. (1992) *Proc. Natl. Acad. Sci. USA* 89: 421-425; Khoury et al. (1992) *J. Exp. Med.* 176: 1355-1364; Karpus and Swanborg (1991) *J. Immunol.* 146: 1163-1168). The effect has been studied most in the rodent model of myelin basic protein (MBP) induced experimental allergic encephalomyelitis. The release of TGF-β and IL-4 from MBP-specific suppressor cells is believed to ameliorate nervous system disease by inhibiting the function of pro-inflammatory cells. IL-10 is another regulatory cytokine that could have such actions. In contrast, the intra-islet cytokine profiles induced by B-chain insulin immunization did not reveal elevations of any of these cytokines, as determined by the present inventors.

Reductions of intra-islet IFN-γ mRNA were repeatedly demonstrated in animals who received insulin B-chain immunizations, but were not observed in mice receiving non-protective therapies. Recently, infiltrating CD8+ lymphocytes have been reported to be the predominant source of IFN-γ in NOD mouse islets. (Anderson et al. (1993) *Autoimmunity* 15: 113-122). We demonstrated that islet cell preparations from B-chain-immunized mice that were depleted of CD4+ lymphocytes had no detectable IFN-γ mRNA. We therefore suggest that immunization with B-chain insulin protects NOD mice from destructive insulitis by reducing CD8+ T lymphocyte effector function. Reduction of IFN-γ may spare β-cells because that cytokine appears to have important effector actions in islet cell autoimmunity. Combined with other cytokines such as TNF-α or IL-1, IFN-γ is cytotoxic to islet β-cells (Rabinovitch et al. (1990) *J. Clin. Endocrinol.* Metab. 71: 151-156; Foulis et al. (1991) *J. Pathol.* 165: 97-103; Campbell et al. (1988) *J. Immunol.* 141: 2325-2329). Furthermore, insulitis and diabetes have been demonstrated in transgenic mice which selectively produced intra-islet IFN-γ under the control of an insulin gene promoter (Sarvetnick et al. (1990) Nature 346: 844-847). Reciprocally, anti-IFN-γ antibody treatments improve disease outcomes. (Campbell et al. (1988) *J. Immunol.* 141: 2325-2329).

If the β-cells can withstand immunological attacks against a limited number of autoantigens, insulin B-chain immunization may abort further progression of the determinant cascade (Kaufman et al. (1993) *Nature* 366: 69-71). It has been suggested that "protective insulitis" arises in islets that become infiltrated by non-pathogenic Th2 CD4+ T lymphocytes rather than by disease-mediating Th1 T cells (Shehadeh et al. (1993) Autoimmunity 6: 291-300; Rapoport et al. (1993) *J. Exp. Med.* 178: 87-99; Harrison et al. (1993) *Lancet* 341: 1365-1369). The adoptive co-transfer experiments described herein at least demonstrate the requirement for CD4+ cells to confer protection after B-chain immunization; albeit the cytokine profiles do not otherwise support this hypothesis. Without wishing to be bound by any particular theory, it is proposed that insulin B-chain immunization stimulates an immune network that induces a protective immune response. The resulting reduction of IFN-γ expression by intraislet CD8+ T lymphocytes appears to be important to the preservation of β-cell function.

The injection of insulin, or insulin fragments, according to the teachings of the subject invention, is distinct from any previous uses of insulin in several critical aspects. For example, it is common practice for individuals with IDD to take daily insulin injections to compensate for the inability of their pancreas to produce sufficient quantities of insulin. By contrast to these daily insulin injections, the procedures of the subject invention would preferably involve a primary immunization followed by periodic boosters. Furthermore, the composition administered according to the subject invention preferably comprises an adjuvant to enhance the immune response. Specifically exemplified herein is the use of incomplete Freund's adjuvant (IFA). Those skilled in the art would appreciate that other adjuvants could be used. Preferred adjuvants are those which stimulate suppressor T cells and/or the Th2 cells; more preferred adjuvants are those which stimulate the suppressive response, and most preferred adjuvants are those which do not stimulate or which depress, the synthesis of interferon-γ.

Another critical distinguishing feature of the subject invention is the use of insulin immunizations before the appearance of clinical symptoms of IDD. Clearly, because of the metabolic activities of insulin, it is not common practice to administer insulin unless there is already clinical evidence of pancreatic islet cell destruction and insulin insufficiency. According to the subject invention, however, insulin, or a fragment thereof, is best administered before any symptoms appear. In a preferred embodiment, only the B chain of insulin is administered. This procedure is highly advantageous because the B chain does not have, by itself, any metabolic function, yet is it able to confer excellent protection against the subsequent onset of IDD. The composition used to elicit the immunoprotective response differs from that used for achieving beta cell rest in that the insulin or portion thereof is combined with adjuvant.

In addition to the use of insulin to immunize against IDD, other antigens specifically associated with the disease may also be used. The principles exemplified herein with respect to diabetes can be utilized to prevent or ameliorate other autoimmune disorders. Specifically, we have found that antigens associated with particular cells known to be susceptible to autoimmune attack can be used to raise a vigorous, but non-destructive, immune response. An unusual and unexpected aspect of the vigorous immune response elicited by the procedures of the subject invention is the accumulation of lymphocytes at the location of the cells known to be the potential target of the destructive immune response. Despite the infiltration of lymphocytes, the targeted cells are protected against the destructive aspects of the immune response.

The procedures of the subject invention are also unique and advantageous in comparison to reports of oral immunization. Most importantly, the parenteral immunization procedure of the subject invention has been found to be highly effective in preventing the onset of IDD. As shown in Figure 1, the parenteral administration of insulin, or the B chain of insulin, results in excellent protection against IDD. Oral immunization has not resulted in such levels of protection. It is also clear that the parenteral procedure results in a markedly different immune response, which may account for the superior results achieved using this procedure. For example, the parenteral procedure using an adjuvant described herein causes a heightened immune response. Clearly, the augmentation of the immune response is counterintuitive as a means for preventing or alleviating a pathological condition caused by overactivity of a component of the immune system. As exemplified by the EAE model, antigen immunization in the past has been used to produce autoimmune disease. By contrast, oral immunization seeks to directly down-regulate (anergize or deplete) the antigen-specific immune response.

The different immune response to the parenteral immunization compared to the response to oral immunization is remarkable. Specifically, excellent protection has been obtained against the progression of autoimmune disease even though there is no reduction in the rate or magnitude of lymphocyte infiltration to the pancreatic islet cell region. Thus, although an immune response still takes place, the pathogenic aspect of this response has been eliminated or overridden. Other modes of insulin administration, such as oral administration or daily parenteral administration of whole insulin without adjuvant, are known to diminish the infiltration of lymphocytes into the islet cell region. Furthermore, the method of the subject invention raises antibodies which are immunoreactive with insulin. Again, this immune response would not, *a priori,* be expected to be beneficial in preventing IDD, and such a stimulation of antibody production is not observed with oral insulin treatments.

Since neither insulin/IFA nor B chain/IFA immunizations by a parenteral route diminished insulitis in contrast to that observed after daily subcutaneous insulin treatments in previous studies, the two protective phenomena appear to involve distinct mechanisms.

Other researchers have attempted antigen-specific therapies in efforts to block the formation of molecular complexes between HLA molecules and T-cell receptors, for example, by saturating the body with a competing antigen. This is expected to dampen the immune response through the prevention of cellular and molecular interactions necessary to initiate and carry on the immune reaction. In contrast to these prior efforts aimed at reducing the immune reaction, the current invention is unexpectedly able to utilize a heightened immune response to specifically interfere with the destruction of self tissue.

For example, intravenous immunization with human GAD₆₅ appears also to be protective in that the onset of diabetes is delayed (Tisch et al. (1993) *Nature* 366: 72-75; Kaufman et al. ibid., pp. 69-72). It has been reported, however, that insulitis was reduced and that the animals were tolerized to GAD₆₅. These results are distinguished from the insulin immunization experiments described herein which do not show decreased insulitis, despite sparing of islet tissue from destruction. Furthermore, the present cell-mediated, active process, as discussed hereinbelow. Antigens useful to vaccinate against autoimmune diseases according to the subject invention can be those involved in the pathogenesis of the particular disease for which vaccination is desired. Other antigens may also be used so long as these antigens are associated with the particular cells or tissues targeted by the autoimmune response, and they are not found in other locations in the body. Preferably, the antigen will be found in abundance in the organ or tissues targeted by the autoimmune disease. Such treatment will localize the immunoregulatory response to the target organ, thereby allowing paracrine actions of the cells induced by the methods taught herein to functionally inactivate the nearby pathogenic cells. In one embodiment of the subject invention, immunization with glucagon is used to protect against diabetes. Although glucagon is associated with the pancreas, it is not involved in the pathogenesis of diabetes. This protective effect could be mediated by inhibitory cytokines such as interleukin-10 and/or TGF-β. Alternatively or additionally, antigens involved in the pathogenic process can raise an immune response and participate in that response in a way which specifically eliminates the destructive aspects of that response.

It should also be noted that, to the extent that 64kDa antigen(s), primarily GAD as previously described, may include antigens other than GAD, the subject invention can be practiced using immunization with these other 64K antigens or fragments thereof. Such fragments may include, for example, the 37 to 40kDa tryptic digest fragments of the 64 kDa protein(s). A 50kDa protein associated with diabetes has also been described.

In a preferred embodiment, individuals potentially at risk to develop IDD are identified by genetic screening, immunological testing, or other diagnostic procedure. The susceptible individual is then immunized with a composition comprising antigens associated with IDD. Preferably, the antigen of choice is expressed only by those cells which are targeted by the autoimmune response or are in the same anatomical location as the cells targeted by the autoimmune response. Preferable, the immunizing composition comprises a suitable antigen and an adjuvant which enhances the regulatory aspect of the immune response. The methodology stimulates an immune response which directs lymphocytes to the anatomical location of the targeted cells and, at the same time, protects the targeted cells from any destructive action of the immune response.

### Materials and Methods

Animals. NOD/Uf mice (Winter [1990] Diabetes 39:975-982) were bred and maintained in a temperature- and light-controlled facility (Department of Pathology, University of Florida). After weaning at 3-4 weeks of age, littermates were housed in groups of no more than 6 per cage. A soy and fishmeal-based chow (Harlan, Madison, WI) and water were available *ad* *libitum.* Typical diabetes rates in the NOD/Uf colony at 40 weeks of age are near 90% in females and 30% in males.

Subcutaneous administration of insulin. Littermates were divided into 3 groups. The first, comprising 23 NOD mice (12 female, 11 male) received 6.25 units (~40 µmoles) of purified porcine isophane insulin (NPH insulin) (Novo Nordisk, Copenhagen), diluted in an equal volume of incomplete Freund's adjuvant (IFA) (Sigma Chemical Company, St. Louis, MO) subcutaneously in multiple injection sites. Complete Freund's adjuvant was avoided because of its known protective effect in NOD mice (Qin et al. (1993) *J. Immunol.* 150: 2072-2080). The second group of 22 mice (10 female, 12 male) was injected with diluent for NPH insulin in IFA and the third group of 23 (12 female, 11 male) received diluent fluid alone. Treatments started at 4 weeks of age and subsequent immunizations were administered at ages 12, 16, and 22 weeks. In a second study, group 1 (13 females, 10 males) and group 2 (12 females, 11 males) mice were treated as the first 2 groups above except that recombinant human NPH insulin (provided by Dr. R. Chance, Eli Lilly, Indianapolis, IN) was substituted in group 1 for the porcine insulin preparation originally used. In addition, 2 new treatments were assessed. Group 3 (12 females, 11 males) and group 4 (13 females, 11 males) received recombinant human A chain insulin (105 µg, 40 µmole) and B chain insulin (140 µg, 40 µmoles) respectively. As in the first study, immunizations were given in IFA commencing at 4 weeks of age, with subsequent injections given at 8 week intervals. In a third NOD mouse experiment, controlled by 18 0.9% saline + IFA-treated littermates (10 female and 8 male), 18 mice (10 female, 8 male) received 250 µg BSA (Sigma) in IFA. A third group of 21 littermates (11 female, 10 male) was treated with 100 µg of the 17 amino acid component peptide of BSA called ABBOS in IFA. This peptide has been postulated to be an environmental molecular mimic of an endogenous islet protein (Karjalainen et al. (1992) supra).

Adoptive transfer. In a representative experiment, thirty female NOD littermates were randomly assigned to receive subcutaneous immunizations with IFA plus either recombinant human A chain (n=9) or B chain (n = 12) insulin, or diluent (n=9) at 4 and 8 weeks of age. At age 10 weeks, splenocyte pools were obtained from these mice, each pool comprising cells from 3 similarly treated animals. These "modulator" splenocytes were mixed with an equal number of pooled splenocytes harvested from 6 untreated, acutely diabetic NOD mice. The splenocytes from the acutely diabetic animals are used to insure that there will be a prompt autoimmune destructive response in nearly all the recipient animals unless the companion cells modulate (i.e., suppress) that response. Generally IDD occurs in recipients about 2-3 weeks after introduction of the acutely diabetic splenocytes if there is no other (modulator) splenocyte population introduced. Six hours after a 700 Rad dose of γ-irradiation, 10 previously untreated, non-diabetic, 10 week old male NOD mice received an intravenous infusion containing a total of 40 x 10⁶ cells from one of the mixtures, i.e. 3 diluent-, 3 A chain-, and 4 B chain-treated animals.

To investigate the roles of CD4+ and CD8+ lymphocytes in the protection conferred by insulin or insulin B chain immunization, donor mice pre-vaccinated with B chain were treated with anti-CD4 or anti-CD8 monoclonal antibodies. Flow cytometry confirmed that these antibody treatments were effective in removing lymphocytes of the target type (see Fig.4A and 4B). Splenocytes from these CD4-ablated or CD8-ablated or control animals were introduced into naive recipients as above, and IDD was monitored.

Diabetes and IAA Monitoring. Venous blood samples, obtained from the tail vein or retroorbital sinus, were tested serially for glucose and IAA. Blood glucose was determined using a Chemstrip bG (Boehringer Mannheim Corp., Indianapolis, IN) read visually as verified by a reflectance meter. Diabetes was diagnosed when the whole blood glucose level was greater than 240 mg/dl (13.3 mmol/L). A liquid phase competitive radiobinding assay using 1:3 diluted mouse sera was used to determine IAA titer (Vardi et al. (1987) *Diabetes* 36: 1286-1291). Normal murine levels of IAA, established from a survey of 48 mice from six different strains that were not susceptible to spontaneous diabetes, were defined herein as below 180 nU/ml (3 standard deviations above mean).

Spleen cell proliferation was assayed by tritiated thymidine incorporation (1µCi/well) during the last 16 hours of an 88 hour incubation. Groups of 3 mice each were immunized at 4 and 8 weeks of age and spleen cells were harvested 1 week later. Using flat-bottomed, 96-well plates, 5 x 10⁵ cells/well were incubated in triplicate with antigen (either insulin A chain or B chain at 20 or 2 µg/ml) in Click's Medium (EHAA) supplemented with 0.5% normal mouse serum (Peck and Bach (1973) *J. Immunol.* Methods 3: 147-164). Cells were harvested, and radioactive uptake was measured using a Matrix 96 automated apparatus (Packard, Meridian, CT). Proliferation studies were performed three times.

Histological Monitoring. Non-diabetic mice in the subcutaneous immunization experiment surviving to 40 weeks were sacrificed at that age. Female NOD mice were immunized to either A-chain (n=9) or B-chain (n=7) insulin or were treated with insulin diluent in IFA (n=9) at 4 and 8 weeks of age, and they were sacrificed at 10 weeks of age, and their pancreata were removed. Their pancreata were removed, fixed in 10% formalin and stained with hematoxylin and eosin for light microscopic examination. The slides were coded, and a consensus insulitis score was determined as described in Atkinson et al. (1990) *Diabetes* 39: 933-937 by three examiners before the code was broken. Pancreatic islets were scored semiquantitatively as 0 when islet cells had no visible signs of inflammation, 1 where the islets had lymphocytes surrounding the islet margin with little or no intraislet infiltration, 2 when islets were surrounded by lymphocytes and contained considerable intraislet inflammation, and 3 when islets were completely engulfed with lymphocytes. Islet destruction was scored as 0 when there was normal islet architecture (nondiabetic), 1 if the islet displayed minor reductions (<25%) in the number of islet β-cells, 2 when most of the islet displayed an atrophied remnant from composed of few residual β-cells and increased relative numbers of non-β-endocrine cells. The inflammation and destruction scores were averaged to provide mean score values. Finally, the pancreata from the modulator cell donors in the adoptive transfer studies were similarly reviewed at 10 weeks of age. Pancreata from 8 of 10 recipients (only 2 of 4 mice receiving B chain modulator cells were sacrificed) were analyzed 20 days after their adoptive transfer.

Cytokine monitoring. Cytokine production within infiltrated islets was measured as follows. At 4 and 8 weeks of age, groups of 3-4 mice each received multiple subcutaneous injections of emulsions of IFA containing either insulin diluent, A-chain, B-chain, or else were left untreated. At 10 weeks of age, islets from non-diabetic mice were hand picked after pancreatic mincing and collagenase/DNAse digestion. The islets were dispersed with Trypsin at 37 °C and the mRNA was extracted using the Micro-Fast Track mRNA Isolation Kit (InVitrogen, San Diego, CA). Cytokine-specific primers were designed as outlined previously (Andersen et al. (1993) supra). After completing RT-PCR and agarose gel electrophoresis, the DNA was Southern blotted to nylon membranes (Boehringer-Mannheim, Indianapolis, IN) in 0.4 N NaOH. Amplimer identities were confirmed using digoxigenin-labeled, cytokine-specific internal probes and the Genius detection system (Boehringer-Mannheim). Since the level of mRNA is dependent on both quantitative and qualitative aspects of the islet infiltrate, the number of islets from which mRNA is extracted is factored into the comparison of band intensity for different mRNA preparations so that relative abundance of a particular message can be compared in different preparations. The studies were performed in duplicate or triplicate.

### In vivo depletion of CD4+ and CD8+ lymphocytes.

Synergistic depleting monoclonal antibody pairs against CD4 (YTS 191.1 and YTA 3.1.2) and CD8 (YTS 169.4 and YTS 156.7) were provided by Dr. Anne Cooke, Cambridge University, U.K. Control mice received injections of either phosphate buffered saline or an IgG monoclonal antibody specific for influenza A nucleoprotein (provided by Dr. Parker Small, University of Florida, Gainesville). Starting 1 week after completing a 2-dose course of IFA/B-chain insulin immunization at 4 and 8 weeks of age, 1 intravenous and then 2 intraperitoneal monoclonal antibody or control treatments were administered. They were given 7, 5, and 3 days before the animals were sacrificed. Flow cytometric analyses of splenocytes from treated or control mice were performed on a FACSCAN (Becton-Dickenson, San Jose, CA) after staining with phycoerythrin-conjugated GK1.5 (anti-CD4) and clone 53-6.7 (anti-CD8) (Pharmingen, San Diego, CA). Using spleen cells from T cell-depleted animals, the B-chain immunization adoptive co-transfer protocol was repeated (Recipients: Anti-CD4 n=6, Anti-CD8 n=6, Control n=7).

### Statistics

The method of Kaplan and Meier (Kaplan and Meier (1958) *J. Am. Stat. Assoc.* 53: 457-481) was used to construct life tables, and the logrank Chi-square statistic was used to compare them (Mantel, N. (1966) *Cancer Chemother. Rep.* 50: 163-170). A Student t-test or one-way ANOVA was used, when appropriate, to compare means. Fisher's exact test was applied to compare frequencies in the adoptive transfer studies. P-values are 2-sided.

All references cited herein are hereby incorporated by reference in their entirety.

Except as noted hereafter, standard techniques for cloning, DNA isolation, amplification and purification, for enzymatic reactions involving DNA ligase, DNA polymerase, restriction endonucleases and the like, and various separation techniques are those known and commonly employed by those skilled in the art. A number of standard techniques are described in Sambrook et al. (1989) Molecular Cloning, Second Edition, Cold Spring Harbor Laboratory, Plainview, New York; Maniatis et al. (1982) Molecular Cloning, Cold Spring Harbor Laboratory, Plainview, New York; Wu (ed.) (1993) *Meth. Enzymol.* 218, Part I; Wu (ed.) (1979) *Meth Enzymol.* 68; Wu et al. (eds.) (1983) *Meth. Enzymol.* 100 and 101; Grossman and Moldave (eds.) *Meth. Enzymol.* 65; Miller (ed.) (1972) Experiments in Molecular Genetics, Cold Spring Harbor Laboratory, Cold Spring Harbor, New York, Old Primrose (1981) Principles of Gene Manipulation , University of California Press, Berkeley; Schleif and Wensink (1982) Practical Methods in Molecular Biology; Glover (ed.) (1985) DNA Cloning Vol. I and II, IRL Press, Oxford, UK; Hames and Higgins (eds.) (1985) Nucleic Acid Hybridization, IRL Press, Oxford, UK; Setlow and Hollaender (1979) Genetic Engineering: Principles and Methods , Vols. 1-4, Plenum Press, New York. Abbreviations and nomenclature, where employed, are deemed standard in the field and commonly used in professional journals such as those cited herein.

Following are examples which illustrate procedures, including the best mode, for practicing the invention. These examples should not be construed as limiting. While various embodiments of the present invention have been described in detail, it is apparent that modifications and adaptations will occur to those skilled in the art. It is to be expressly understood that such modifications and adaptations are within the spirit and scope of the present invention, as set forth in the following claims. All percentages are by weight and all solvent mixture proportions are by volume unless otherwise noted.

### THE EXAMPLES

### Example 1 - The Effect of Subcutaneous Insulin

Logrank analysis showed that subcutaneous insulin in IFA significantly reduced the incidence of diabetes in NOD mice (p=.0002). The mice receiving diluent alone or diluent + IFA developed IDD at similar rates (p = .3) to those typical for the NOD/Uf colony. When compared to animals treated with diluent in IFA, immunization of animals with the B chain component of insulin in IFA resulted in protection from diabetes. Immunization with A chain insulin did not, however, alter the course of IDD. Importantly, the life table curves of the animals immunized with either BSA or ABBOS peptide were not different from those of their saline-treated controls. Since the 2 slightly different immunization protocols yielded similar results, pooled data are presented. As shown in figure 1, insulin immunizations dramatically lowered the incidence of diabetes over the 40 week follow-up period. The protection was reproduced by immunizations with insulin B-chain, but importantly not with treatments of A-chain insulin or bovine serum albumin.

An increase in the incidence and persistence of antibodies to insulin was observed in those animals receiving insulin immunizations. The treatments stimulated antigen-specific responses in both the humoral and the cellular arms of the immune system. At 12 weeks of age, only 3 or 21 diluent treated and 3 of 23 diluent + IFA treated mice had positive IAA levels detected, while 12 of 21 injected with insulin were positive for insulin antibodies as detected by the RIA binding method (p<.001). At 26 weeks, insulin antibodies persisted only in the insulin-immunized mice. Both the A and B chain immunizations stimulated high levels of insulin antibodies.

Figure 2A presents the frequency of antibody-positive mice after immunization. For the diluent- and IFA-treated mice, there was a low frequency of animals positive for insulin-specific antibodies (insulin autoantibodies) of relatively low titer. In the insulin-immunized mice, specific antibodies were detected with significantly higher frequencies and at titers that were at least tenfold above those typical for insulin autoantibodies in untreated NOD mice. Similarly, increases of insulin binding antibodies were also observed in sera from mice immunized against either A- or B-chain insulin. Antigen-specific proliferative responses measured *in vitro*, while weak, were consistently stimulated as demonstrated in Figure 2B.

Figure 1 shows extended protection from IDD in mice immunized with either insulin or B chain insulin at 4 and 8 weeks. These animals received the vaccinations before NOD mice typically display overt signs of islet cell destruction. Figure 7 shows protection afforded by insulin B chain administered to NOD mice at 10 weeks of age. Significant protection is observed at least up to 50 weeks of age. These results show that it is not essential that the adjuvant + insulin vaccinations be given before the autoimmune destruction of the target issue has begun, but rather that stimulation of the suppressor T cells (or antigen-specific T cells which are capable of down-regulating the destructive response of cells in the vicinity) can slow or stop an autoimmune process already in progress.

Pancreatic histologies were not different among the mice who survived to 40 weeks without developing diabetes, regardless of the experimental treatment group.

### Example 2 - Adoptive Transfer of B Chain-Induced Protection

Protection from diabetes can be adoptively transferred to naive recipients. As presented in Table 2, diabetes was transferred within 20 days to 10 week old, irradiated, male NOD recipients by splenocytes from diabetic NOD mice, co-administered with an equal number of splenocytes from animals that had been treated by 2 prior immunizations of diluent (3/3) of A chain (3/3) in IFA. In contrast, diabetes was prevented for at least 75 days (p=0.3) in similar recipients that received co-transfers of splenocytes from IFA/B chain treated together with untreated, acutely diabetic mice. The insulitis scores in mice donating splenocytes as well as in recipient mice sacrificed 20 days after transfer were not different among the 3 treatment groups.

**Table 2.**

| B chain immunization prevents adoptive transfer of IDD | | | |
|---|---|---|---|
| Treatment | Number of Diabetic Mice | | |
| | Day 10 | Day 20 | Day 75 |
| Diluent | 0/3 | 3/3 | - |
| A Chain | 0/3 | 3/3 | - |
| B Chain | 0/4 | 0/4* | 0/2** |

| | | | |
|---|---|---|---|
| *p = 0.03, B chain vs. diluent or B chain vs. A chain | | | |
| ** B chain-treated mice were sacrificed on day 20 for histological studies. | | | |

Protection from diabetes can be adoptively transferred to naive recipients. To confirm the active immune basis of the therapeutic effect, 10 week old male, irradiated recipient NOD mice were injected with spleen cells from immunized female littermates plus an equal number of splenocytes harvested from diabetic co-donors. Among recipients of splenocytes from the untreated (n=3), A-chain-immunized (n=3), and diluent-immunized control groups (n=8), 11/14 (79%) became diabetic within 3 weeks of cell transfer. In contrast, diabetes had occurred in only 3 of 15 (20%) recipients of splenocytes from B-chain-immunized donors (p<0.007) (Figure 3). Thus, B-immune splenocytes can stop the autoimmune destruction by the co-transferred IDD splenocytes. These results indicate that the immune modulator cells stimulated by insulin B chain determinants, can down regulate destructive lymphocytes, presumably having a variety of antigenic specificities, consistent with a "bystander immunosuppression" phenomenon.

In mice that received a course of lymphocyte-depleting antibodies, flow cytometric analysis of splenocytes revealed nearly complete depletion by the monoclonal pairs (anti-CD4:94%, anti-CD8:96%) compared to PBS injected, B-chain immunized mice (Figs. 4A and 4B). *In vivo* depletion of either CD4+ or CD8+ T lymphocytes before transfer abrogated the ability of B-chain-immunized splenocytes to prevent the transfer of diabetes (p<0.01). Three weeks after the splenocyte transfer, 5 of 6 recipients depleted of CD4+ cells and 4 of 5 recipients depleted of CD8+ cells became hyperglycemic (Figure 5). These experiments suggest that an active antigen-specific immunoregulatory lymphocyte network, promoted by B-chain immunizations, can inhibit the autoimmunity that normally causes islet cell destruction, and that both CD4+ and CD8+ cell types are required to produce the protective response.

The adoptive co-transfer experiments demonstrate that the transferred protection is not permanent; it persisted about 8 weeks in these experiments. The experiments further demonstrate that the protective process (or suppressive, relative to immune-mediated tissue destruction) is cell-mediated, and that the protective process is an active one. The active nature of the protective response afforded by insulin vaccination is distinguished from tolerization and/or anergy because tolerization/anergy is a passive process-administration of antigen saturates available binding sites and prevents further damage. This type of protection is not transmissible from one animal to another via splenocytes as is the subject process. Because in the transferred protective response, the "B-immunized" cells suppress the destruction of pancreatic islet cells by the splenocytes of the acutely diabetic IDD mice, clearly the suppressive/protective process is active and involves B-chain-specific immune cells capable of negatively regulating even immune cells of other specificities to spare the target tissue.

Histological insulitis grade is not decreased by B-chain insulin immunization. Despite the lower frequency of diabetes in B-chain-immunized mice, histologic studies revealed that the extent of mononuclear leukocyte infiltration of islets was not reduced. Using an infiltration scale as described hereinabove and by Atkinson et al. (1990) supra, the mean and standard error insulitis scores in pancreata of 10 week old mice, immunized at 4 weeks of age, were 0.9± 0.2, 1.3 ± 0.3, and 1.5 ± 0.2 for diluent (n=0 mice), A-chain (n=9 mice) and B-chain (n=7 mice) treated groups respectively (p=0.3). Further, flow cytometric analyses revealed no differences in the proportions of CD3+, CD4+, CD8+, or 1-A+ cells in dispersed islet cell preparations from treated and control mice.

Immunizations alter cytokine production within infiltrated islets. To assess whether functional differences exist between the islet-infiltrating immune cells of treated and control mice, cytokine mRNA profiles were examined by semi-quantitative RT-PCR analyses (Andersen et al. (1993) supra). The panel of cytokine-specific primers included interleukin (IL)-1β, IL-2, IL-4, IL-10, transforming growth factor (TGF)-β₃, and interferon (IFN)-γ. The salient results are presented in Figure 6A-6D. Amplimer specific to particular cytokine messages were prepared from nondiabetic (NOD) mouse pancreatic islets. High levels of I1-10 and TGF-β₃ were observed in untreated mice but were not consistently increased further by insulin immunizations. Increased levels of IL-4 mRNA were observed after immunizations with IFA alone, an effect which was reversed after immunizations by either of the insulin peptide chains. Immunizations with either A-chain or B-chain increased intra-islet levels of IL-1β and IL-2 mRNA compared to controls. Intra-islet levels of IFN-γ message appeared to be increased by immunization with insulin A chain (with IFA). However, the most notable change, specific to islet infiltrates of the highly protected B-chain-immunized mice, was a consistently reduced level of IFN-γ mRNA. Depletion of CD4+ cells in B-chain insulin-immunized mice further reduced the levels of IFN-γ below the limits of detection, while the reduced levels of cytokine transcript persisted in animals depleted of their CD8+ T lymphocytes (Figure 6D, right-most panel). This indicated a marked reduction in the secretion of IFN-γ by CD8+ T cells after insulin B chain immunization. It is noted that because both CD4+ and CD8+ T cells were required in the adoptive co-transfer experiments, the decrease in IFN-γ expression cannot alone account for the protective effect of B chain determinants.

### Example 3 - Vaccine Compositions

Vaccines for immunizing against an autoimmune condition, in accordance with the teachings of the subject invention, can be prepared by procedures well known in the art. For example, such vaccines can be prepared as injectables, e.g., liquid solutions or suspensions. Solid forms for solution in, or suspension in, a liquid prior to injection also can be prepared. Optionally, the preparation also can be emulsified. The active antigenic ingredient or ingredients can be mixed with excipients which are pharmaceutically acceptable and compatible with the active ingredient. Examples of suitable excipients are water, saline, dextrose, glycerol, ethanol, or the like, and combinations thereof.

In addition, if desired, the vaccine can contain minor amounts of auxiliary substances such as wetting or emulsifying agents, pH buffering agents, and/or adjuvants which enhance the effectiveness of the vaccine. In a preferred embodiment, adjuvants such as Freund's adjuvant, Freund's incomplete adjuvant, aluminum hydroxide, alum or muramyl dipeptide or variations thereof are used. Other adjuvants known to the art may be substituted. Also, cholera toxin subunit B or other agents which stimulate antibody production at mucosal sites can be used. When peptides are used to raise the immune response, the peptides may be coupled to larger molecules such as keyhole limpet hemocyanin or tetanus toxoid using art-known techniques to enhance immunogenicity. The vaccines are conventionally administered parenterally, by injection, for example, either subcutaneously or intramuscularly.

An adjuvant suitable for use in vaccine compositions in combination with a target antigen of an autoimmune disease is one which stimulates humoral immunity rather than cell-mediated immunity, and/or one which elicits an immunosuppressive response which will protect the target tissue from autoimmune destruction. As exemplified herein, incomplete Freund's adjuvant is combined with insulin or B chain insulin. According to the product description in the 1994 Sigma Chemical Company catalog, IFA is an 85:15 (volume:volume) mix of paraffin oil and mannide monooleate. IFA can be replaced with nontoxic, preferably metabolizable oil adjuvants known to the art such as soybean oil or sesame oil or other adjuvants such as alum/aluminum salts, acemannan and others. Where oils are used, it may be desired to include an emulsifying agent to the vaccine composition, choosing from those well known. Suitable adjuvants for use in the present invention can be selected by the skilled artisan using the present disclosure and the art known properties of adjuvants, for example, as reviewed by Warren et al. (1986) *Ann. Rev. Immunology* 4:369-388. Preferably, the vaccine composition is tested as taught herein to confirm that its administration prevents, delays the onset, or lessens the symptoms of the autoimmune disease and the destruction of the target tissue is prevented or lessened, which can be monitored by interferon-γ levels, for example.

The compounds can be formulated into the vaccine as neutral or salt forms. Pharmaceutically acceptable salts include the acid addition salts (formed with the free amino groups of the protein or peptide) and which are formed with inorganic acids such as, for example, hydrochloric or phosphoric acids, or such organic acids as acetic, oxalic, tartaric, mandelic, maleic and the like. Salts formed with the free carboxyl groups can also be derived from inorganic bases such as, for example, sodium, potassium, ammonium, calcium, or ferric hydroxides, and such organic bases as isopropylamine, trimethylamine, 2-ethylamino-ethanol, histidine, procaine, and the like.

Immunogenic carriers may be used to enhance the immunogenicity of the proteinases. Such carriers include but are not limited to proteins and polysaccharides, liposomes, and bacterial cells and membranes. Protein carriers may be joined to the antigens to form fusion proteins by recombinant or synthetic means or by chemical coupling. Useful carriers and means of coupling such carriers to polypeptide antigens are known in the art.

The vaccines may be formulated by any of the means known in the art. Such vaccines are typically prepared as injectables, either as liquid solutions or suspensions. Solid forms suitable for solution in, or suspension in, liquid prior to injection may also be prepared. The preparation may also, for example, be emulsified, or the protein encapsulated in liposomes.

In addition, if desired, the vaccines may contain minor amounts of auxiliary substances such as wetting or emulsifying agents, pH buffering agents, and/or adjuvants which enhance the effectiveness of the vaccine. Examples of adjuvants which may be effective include but are not limited to: alum, poly (DL-lactide-co-glycolide), MF59(squalene, polysorbate 80, sorbitan trioleate; Ciba Geigy), aluminum hydroxide; N-acetyl-muramyl-L-threonyl-D-isoglutamine; N-acetyl-nor-muramyl-L-alanyl-D-isoglutamine (CGP 11637, also referred to as nor-MDP); N-acetylmuramyl-L-alanyl-D-isoglutaminyl-L-alanine-2-(1'-2'-dipalmitoyl-sn-glycero-3-hydroxyphosphoryloxy)-ethylamine (CGP 19835A, referred to as MTP-PE); and RIBI, which contains three components extracted from bacteria, monophosphoryl lipid A, trehalose dimycolate and cell wall skeleton in 2% squalene-Tween 80 emulsion. The effectiveness of an adjuvant may be determined by measuring the amount of antibodies directed against the immunogen resulting from administration of the immunogen in vaccines which are also comprised of the various adjuvants. Such additional formulations and modes of administration as are known in the art may also be used.

The vaccines are administered in a manner compatible with the dosage formulation, and in such amount as will be therapeutically effective and immunogenic. The quantity to be administered is generally in the range of about 100 to 1,000 µg of protein per dose, more generally in the range of about 5 to 500 µg of protein per dose. The quantity to be administered can depend on the subject to be treated and the degree of protection desired. Precise amounts of active ingredient required to be administered depend on the judgment of the practitioner and can be peculiar to each individual. However, suitable dosage ranges are of the order of about several hundred micrograms active ingredient per individual. Suitable regimes for initial administration and booster shots are also variable, but are typified by an initial administration followed in one or two week intervals by a subsequent injection or other administration.

### Example 4 - Viral Vaccines

The immunizing compositions of the subject invention may also be administered using a live viral vaccine. Such vaccines are known in the art and are particularly advantageous in delivering an antigen in prolonged manner. Viral vaccines typically comprise a non-pathogenic virus which has had a heterologous gene, coding for a particular antigen, inserted into the viral genome. As the virus replicates inside the vaccinated host, the foreign antigen is expressed. The host then mounts the desired immune response against the foreign antigen.

The choice of a suitable virus from which to construct a vaccine can be made by a person skilled in the art. Studies with vaccinia virus have demonstrated that poxviruses in general have several advantageous features as vaccine vectors. Poxviruses are taxonomically classified into the family Chordopoxvirinae, whose members infect vertebrate hosts, e.g., the Orthopoxvirus vaccinia. Vaccinia virus has been developed as a eukaryotic cloning and expression vector (Mackett et al., (1985) *DNA Cloning,* Vol. II, ed. D.M. Glover, pp. 191-212, Oxford: IRL Press; Panicali et al., (1982) *Proc. Natl. Acad. Sci. USA* 88: 5364-5368). Numerous viral antigens have been expressed using vaccinia virus vectors (Paoletti et al., (1986) *Proc. Natl. Acad. Sci. USA* 81: 193-197; Piccine et al., (1986) *BioEssays* 5: 248-252) including, among others, HBsAg, rabies G protein and the gp120/gp41 of human immunodeficiency virus (HIV). Regulatory sequences from the spruce budworm EPV have been used previously with vaccinia (Yuen et al. (1990) *Virology* 175: 427-433).

Preferably, a DNA segment encoding an insulin B chain is cloned into a vaccinia vector chosen from those known in the art useful in humans, or other mammals desired to be protected against IDD, with the cloning performed so as to result in the expression of the insulin B chain, according to art-known principles.

Exemplary insulin B chains have amino acid sequences given below as SEQ ID NO:3 and 4. The skilled artisan knows how to design coding sequences suitable for expression of B chain peptides, or he may utilize the known amino acid or nucleotide sequences to express insulin or proinsulin polypeptides, which sequences are well known to the art.

Bovine and human insulin B chains have the amino acid sequence phe-val-asn-gln-his-leu-cys-gly-ser-his-leu-val-glu-ala-leu-tyr-leu-val-cys-gly-glu-arg-gly-phe-phe-tyr-thr-pro-lys-thr (SEQ ID NO:3).

Porcine B chain has the amino acid sequence phe-val-asn-gln-his-leu-cys-gly-ser-his-leu-val-glu-ala-leu-tyr-leu-val-cys-gly-glu-arg-gly-phe-phe-tyr-thr-pro-lys-ala (SEQ ID NO:4).

The human nucleotide sequence encoding human proinsulin and preproinsulin is given, e.g., in U.S. Patent No. 4,431,740 (Bell et al., issued February 14, 1984), incorporated by reference herein.

Alternatively, recombinant vectors can be constructed using the nucleotide sequences encoding human GAD₆₅ from pancreatic tissue, as described in Yamashita et al. (1993) *Biochem. Biophys. Res. Comm.* 192: 1347-1352; Kawasaki et al. (1993) ibid, pp. 1353-1359. Mauch et al. (1993) *Eur. J. Biochem.* 212: 597-603 describes a linear epitope within pancreatic GAD₆₄ and its recognition by IDD sera. Grubin et al. (1992) *Clin. Res.* 40(2): 299A refers to a GAD nascent peptide autoantigen of IDD. The skilled artisan can adapt the methods described herein to confirm that use of the peptides of these references can be used to delay or prevent the onset of IDD.

The advantages of poxviruses as vaccine vectors include the ability of poxvirus-based vaccines to stimulate both cell-mediated and humoral immunity, minimal cost to mass produce vaccine and the stability of the lyophilized vaccine without refrigeration, ease of administration under non-sterile conditions, and the ability to insert at least 25,000 base pairs of foreign DNA into an infectious recombinant, thereby permitting the simultaneous expression of many antigens from one recombinant.

Viral vaccine formulations may contain appropriate, conventional carriers or diluents, preservatives, pH adjusters or stabilizers. For example, suitable carriers and diluents include sterile saline, lactose, sucrose, calcium phosphate, gelatin, dextrin, agar, pectin, peanut oil, olive oil, sesame oil and water. Additionally, the carrier or diluent may include a time delay material, such as glycerol monostearate or glycerol distearate alone or with a wax. In addition, slow release polymer formulations known to the art can be used.

A therapeutic composition or vaccine composition of the invention may contain between about 10¹ to about 10⁸ of the recombinant virus per dose. These compositions contain suitable amounts of the active ingredient, the recombinant viral vector containing the antigen, which can be determined by one of skill in the art based upon the level of immune response desired. In general, however, the therapeutic or vaccine composition contains between about 1 x 10⁵ to about 1 x 10⁷ plaque forming units (PFU) per mL, and more preferably between 1 x 10⁶ and 3 x 10⁶ PFU/mL.

The therapeutic or vaccine composition of the invention may contain one or more antigens directed to the same or different autoimmune diseases.

A viral vaccine composition of the invention may also contain a suitable adjuvant. The adjuvant is used as a nonspecific irritant to attract leukocytes or enhance an immune response. Such adjuvants include, among others, mineral oil and water, aluminum hydroxide, Amphigen, Avridine, L121/squalane, D-lactide-polylactide/glycoside, pluronic plyois, muramyl dipeptide, killed Bordetella, saponins, as Quil A.

Suitable doses of the vaccine composition of the invention can be readily determined by one of skill in the art. Generally, a suitable dose is between 1 to 5 mL of the vaccine composition. Further, the quantity to be administered depends on the size of the host to be treated, the capacity of the host's immune system to synthesize antibodies, the degree of protection desired, and may be adjusted by one of skill in the art. However, suitable dose ranges are of the order of from about one hundred to about several hundred micrograms of active ingredient per host.

The therapeutic or vaccine compositions are administered in a manner compatible with the dosage formulation, and such amount as will be therapeutically effective and immunogenic. Typically, the composition can be administered by intradermal scarification using a bifurcated needle similar to that used for smallpox vaccines. However, any suitable route is acceptable. Preferably, this route is intradermal, intramuscular, or subcutaneous. It is anticipated that a suitable regime for administration is a single treatment.

### Example 5 - BCG Vaccine

An additional vaccination procedure useful according to the subject invention can utilize recombinant attenuated Mycobacterium tuberculin vaccine (BCG) to provide the vehicle for vaccination. As recognized by those skilled in the art, tuberculin can augment the formation of Th2 (T helper 2) cells which secrete interleukins 4 and 10 and TGF-β and augment B cell antibody secretion. These properties can be used advantageously to enhance the creation of antigen-specific regulatory T cells to override the autoimmune process and thereby induce protection.

Preferably, a DNA segment encoding an insulin B chain is cloned into a vector chosen from those known in the art, with the cloning performed so as to result in the expression of the insulin B chain, according to art-known principles. Cloning vehicles for introduction and expression of recombinant DNA in M. bovis BCG are described, for example, in Andrew et al. (1993) *J. Clin. Microbiol.* 31: 2251-2254; Murray et al. (1992) *Molec. Microbiol.* 6: 3331-3342; Aldovini et al. (1993) *J. Bacteriol.* 176: 419-425; Goto et al. (1991) *FEMS Microbiol. Lett.* 83: 277-282.

Exemplary insulin B chains have amino acid sequences given below as SEQ ID NO:3 and 4. The skilled artisan knows how to design coding sequences suitable for expression of B chain peptides, or he may utilize the known amino acid or nucleotide sequences to express insulin or proinsulin polypeptides, which sequences are well known to the art.

Bovine and human insulin B chains have the amino acid sequence phe-val-asn-gln-his-leu-cys-gly-ser-his-leu-val-glu-ala-leu-tyr-leu-val-cys-gly-glu-arg-gly-phe-phe-tyr-thr-pro-lys-thr (SEQ ID NO:3).

Porcine B chain has the amino acid sequence phe-val-asn-gln-his-leu-cys-gly-ser-his-leu-val-glu-ala-leu-tyr-leu-val-cys-gly-glu-arg-gly-phe-phe-tyr-thr-pro-lys-ala (SEQ ID NO:4).

The human nucleotide sequence encoding human proinsulin and preproinsulin is given, e.g., in U.S. Patent No. 4,431,740 (Bell et al., issued February 14, 1984), incorporated by reference herein.

Alternatively, recombinant vectors can be constructed using the nucleotide sequences encoding human GAD₆₅ from pancreatic tissue, as described in Yamashita et al. (1993) *Biochem. Biophys. Res. Comm.* 192: 1347-1352; Kawasaki et al. (1993) ibid, pp. 1353-1359. Mauch et al. (1993) *Eur. J. Biochem.* 212: 597-603 describes a linear epitope within pancreatic GAD₆₄ and its recognition by IDD sera. Grubin et al. (1992) *Clin. Res.* 40(2): 299A refers to a GAD nascent peptide autoantigen of IDD. The skilled artisan can adapt the methods described herein to confirm that use of the peptides of these references can be used to delay or prevent the onset of IDD.

### Example 6 - Vaccination Programs

Vaccination can be carried out according to the subject invention to provide protection to the general public or to particular individuals believed to be at risk for developing a specific autoimmune disease. For example, in the case of diabetes, children can be screened genetically and through the use of biochemical markers to determine with considerable accuracy the likelihood of a particular child ultimately developing diabetes. Specifically, the prevalence of IDD in the United States is 1 in 300 over a lifetime. Among first degree relatives of affected patients, IDD afflicts an additional 6-10%, depending on age. One in every 3 identical twin pairs affected by IDD become concordant for the disease. It is also known that one major gene involved in this evident inherited susceptibility is that located at the HLA-DQ locus. DQ human leukocyte antigens, encoded by the short arm of chromosome 6, are heterodimeric molecules which have importance to immunoresponsiveness to specific antigens. Genetic tests are being developed to provide highly accurate information regarding the likelihood of developing IDD. Also, it is well known that persons who have just developed IDD are in process of developing IDD have a number of disease-specific autoantibodies in their blood. Such autoantibodies include those to islet cell antigens (ICA), to beta cell specific proteins of 64 dDa (now thought to be the lower molecular isoform of glutamic acid decarboxylase [GAD₆₅]) and of 38 kDa, to native insulin and proinsulin, and to a number of more minor determinants such as carboxypeptidase-H and heat shock proteins belonging to the hsp-60 family. Among unaffected relatives of patients with IDD, the five year predictive value of insulin autoantibodies (IAA) is near 10%, that of ICA approximate 25%, while those having both antibodies have a risk approaching 70% (Riley et al. (1990) *New Engl. J. Med.* 323: 1167-1172; Bonifacio et al. (1990) *Lancet* 335: 147-149; Krischer et al. (1993) *J. Clin. Endo. Metab.* 77: 743-749). Greater predictive risks are associated with antibodies to the 64 kDa antigen (Atkinson et al. (1990) *Lancet* 335: 1357-1360). Additional risk can be identified for antibody positive persons who develop an impaired ability to secrete insulin within 3 minutes following a standard intravenous glucose challenge (Robert et al. (1991) *Diabetes Care* 14: 718-723). These same antibodies can be used to identify those children at risk by screening a general population (Maclaren et al. (1985) *Diabetes* 34: 97A). This is important because approximately 85% of all patients with IDD know of no other affected relative.

These accurate means of predicting IDD susceptibility can be used as a basis for determining who should be vaccinated according to the subject invention. Additionally, because of the great risk presented by IDD, a vaccination program to immunize all children at an early age can be instituted.

It should be understood that the examples and embodiments described herein are for illustrative purposes only and that various modifications or changes in light thereof will be suggested to persons skilled in the art and are to be included within the spirit and purview of this application and the scope of the appended claims.

## Claims

1. The use of an adjuvant and an antigen in the preparation of a composition useful in the suppression of immune-mediated destruction of pancreatic tissue in a mammal suffering from or susceptible to insulin dependent diabetes, said antigen being the B chain of insulin or a fragment thereof, and said composition being adapted for administration by parenteral injection.

2. The use according to claim 1 wherein the antigen is a peptide comprising an amino acid sequence as shown in SEQ ID NO:1, SEQ ID NO:2, SEQ ID NO:3, or SEQ ID NO:4.

3. The use according to claim 1 wherein the result of the administration of the composition is the decreased expression of interferon-γ by CD8+ T lymphocytes within said pancreatic tissue.

4. The use according to any of claims 1-3 wherein said adjuvant is Freund's incomplete adjuvant.

5. The use according to any of claims 1-4 wherein said antigen is an expression product of a recombinant viral vector.

6. The use according to any of claims 1-4 wherein said antigen is an expression product of a recombinant *Mycobacterium bovis* BCG strain.

7. A composition for suppressing immunologically mediated destruction of pancreatic tissue in a mammal suffering from or susceptible to insulin dependent diabetes, said composition comprising an antigen and an adjuvant in a pharmaceutical carrier adapted for parenteral administration, wherein said antigen is the B chain of insulin or a fragment thereof.

8. A composition according to claim 7 wherein said antigen is a peptide comprising an amino acid sequence as shown in SEQ ID NO:1, SEQ ID NO:2, SEQ ID NO:3, or SEQ ID NO:4.

9. The composition according to claim 8 wherein said adjuvant is Freund's incomplete adjuvant.

10. A composition for suppressing immunologically mediated destruction of pancreatic tissue in a mammal suffering from or susceptible to insulin dependent diabetes, said composition comprising a recombinant virus, or a recombinant *Mycobacterium bovis* BCG comprising a heterologous nucleotide sequence encoding an antigen, wherein said antigen is the B chain of insulin or a fragment thereof.

11. A composition according to claim 10, wherein said antigen is a peptide comprising an amino acid sequence as shown in SEQ ID NO:1, SEQ ID NO:2, SEQ ID NO:3, or SEQ ID NO:4.

12. The composition according to claim 10 or claim 11 wherein said recombinant virus is a recombinant *Vaccinia* virus.

13. The use according to claim 2, wherein the peptide is conjugated to a larger molecule.

14. The use according to claim 13 wherein the larger molecule is keyhole limpet hemocyanin or tetanus toxoid.

15. The composition according to claim 8, wherein the peptide is conjugated to a larger molecule.

16. The composition according to claim 15 wherein the larger molecule is keyhole limpet hemocyanin or tetanus toxoid.

## Patentansprüche

1. Verwendung eines Adjuvans und eines Antigens für die Herstellung einer Zusammensetzung, die zur Unterdrückung einer Immun-vermittelten Zerstörung von Pankreasgewebe in einem Säuger geeignet ist, der an einer Insulin-abhängigen Diabetes leidet oder dafür empfänglich ist, wobei das Antigen die B-Kette von Insulin oder ein Fragment davon ist, und wobei die Zusammensetzung für eine Verabreichung durch eine parenterale Injektion angepaßt ist.

2. Verwendung nach Anspruch 1, wobei das Antigen ein Peptid ist, umfassend eine Aminosäuresequenz, wie in SEQ ID NO:1, SEQ ID NO:2, SEQ ID NO:3 oder SEQ ID NO:4 gezeigt.

3. Verwendung nach Anspruch 1, wobei das Ergebnis der Verabreichung der Zusammensetzung die verminderte Expression von Interferon-γ durch CD8+T-Lymphozyten innerhalb des Pankreasgewebes ist.

4. Verwendung nach einem der Ansprüche 1 bis 3, wobei das Adjuvans Freund's unvollständiges Adjuvans ist.

5. Verwendung nach einem der Ansprüche 1 bis 4, wobei das Antigen ein Expressionsprodukt eines rekombinanten viralen Vektors ist.

6. Verwendung nach einem der Ansprüche 1 bis 4, wobei das Antigen ein Expressionsprodukt eines rekombinanten *Mycobacterium bovis*-BCG-Stamms ist.

7. Zusammensetzung zum Unterdrücken der immunologisch vermittelten Zerstörung von Pankreasgewebe in einem Säuger, der an einer Insulinabhängigen Diabetes leidet oder dafür empfänglich ist, wobei die Zusammensetzung ein Antigen und ein Adjuvans in einem pharmazeutischen Träger umfaßt, angepaßt für die parenterale Verabreichung, wobei das Antigen die B-Kette von Insulin oder ein Fragment davon ist.

8. Zusammensetzung nach Anspruch 7, wobei das Antigen ein Peptid ist, umfassend eine Aminosäuresequenz, wie in SEQ ID NO:1, SEQ ID NO:2, SEQ ID NO:3 oder SEQ ID NO:4 gezeigt.

9. Zusammensetzung nach Anspruch 8, wobei das Adjuvans Freund's unvollständiges Adjuvans ist.

10. Zusammensetzung zum Unterdrücken der immunologisch vermittelten Zerstörung von Pankreasgewebe in einem Säuger, der unter einer Insulinabhängigen Diabetes leidet oder dafür empfänglich ist, wobei die Zusammensetzung einen rekombinanten Virus oder einen rekombinanten *Mycobacterium bovis*-BCG umfaßt, umfassend eine heterologe Nukleotidsequenz, die ein Antigen kodiert, wobei das Antigen die B-Kette von Insulin oder ein Fragment davon ist.

11. Zusammensetzung nach Anspruch 10, wobei das Antigen ein Peptid ist, umfassend eine Aminosäuresequenz, wie in SEQ ID NO:1, SEQ ID NO:2, SEQ ID NO:3 oder SEQ ID NO:4 gezeigt.

12. Zusammensetzung nach Anspruch 10 oder 11, wobei der rekombinante Virus ein rekombinanter *Vaccinia*-Virus ist.

13. Verwendung nach Anspruch 2, wobei das Peptid an ein größeres Molekül konjugiert ist.

14. Verwendung nach Anspruch 13, wobei das größere Molekül Keyhole-Limpet-Hämocyanin oder Tetanustoxoid ist.

15. Zusammensetzung nach Anspruch 8, wobei das Peptid an ein größeres Molekül konjugiert ist.

16. Zusammensetzung nach Anspruch 15, wobei das größere Molekül Keyhole-Limpet-Hämocyanin oder Tetanustoxoid ist.

## Revendications

1. Utilisation d'un adjuvant et d'un antigène dans la préparation d'une composition utile pour la suppression de la destruction à médiation immunologique du tissu pancréatique chez un mammifère souffrant de diabète insulino-dépendant ou prédisposé à cette maladie, ledit antigène étant la chaîne B de l'insuline ou un fragment de celle-ci, et ladite composition étant adaptée pour une administration par injection parentérale.

2. Utilisation selon la revendication 1, dans laquelle l'antigène est un peptide comprenant une séquence d'acides aminés telle que présentée dans SEQ ID NO:1, SEQ ID NO:2, SEQ ID NO:3 ou SEQ ID NO:4.

3. Utilisation selon la revendication 1, dans laquelle le résultat de l'administration de la composition est la diminution de l'expression de l'interféron-γ par les lymphocytes T CD8+ dans ledit tissu pancréatique.

4. Utilisation selon l'une quelconque des revendications 1-3, dans laquelle ledit adjuvant est l'adjuvant incomplet de Freund.

5. Utilisation selon l'une quelconque des revendications 1-4, dans laquelle ledit antigène est un produit d'expression d'un vecteur viral recombinant.

6. Utilisation selon l'une quelconque des revendications 1-4, dans laquelle ledit antigène est un produit d'expression d'une souche recombinante de BCG *Mycobacterium bovis.*

7. Composition permettant de supprimer la destruction à médiation immunologique du tissu pancréatique chez un mammifère souffrant de diabète insulino-dépendant ou prédisposé à cette maladie, ladite composition comprenant un antigène et un adjuvant dans un véhicule pharmaceutique adapté pour l'administration parentérale, dans laquelle ledit antigène est la chaîne B de l'insuline ou un fragment de celle-ci.

8. Composition selon la revendication 7, dans laquelle ledit antigène est un peptide comprenant une séquence d'acides aminés telle que présentée dans SEQ ID NO:1, SEQ ID NO:2, SEQ ID NO:3 ou SEQ ID NO:4.

9. Composition selon la revendication 8, dans laquelle ledit adjuvant est l'adjuvant incomplet de Freund.

10. Composition permettant de supprimer la destruction à médiation immunologique du tissu pancréatique chez un mammifère souffrant de diabète insulino-dépendant ou prédisposé à cette maladie, ladite composition comprenant un virus recombinant, ou un BCG *Mycobacterium bovis* recombinant comprenant une séquence nucléotidique hétérologue codant pour un antigène, ledit antigène étant la chaîne B de l'insuline ou un fragment de celle-ci.

11. Composition selon la revendication 10, dans laquelle ledit antigène est un peptide comprenant une séquence d'acides aminés telle que présentée dans SEQ ID NO:1, SEQ ID NO:2, SEQ ID NO:3 ou SEQ ID NO:4.

12. Composition selon la revendication 10 ou la revendication 11, dans laquelle ledit virus recombinant est un virus *Vaccinia* recombinant.

13. Utilisation selon la revendication 2, dans laquelle le peptide est conjugué à une molécule plus grosse.

14. Utilisation selon la revendication 13, dans laquelle la molécule plus grosse est l'hémocyanine de patelle ou l'anatoxine tétanique.

15. Composition selon la revendication 8, dans laquelle le peptide est conjugué à une molécule plus grosse.

16. Composition selon la revendication 15, dans laquelle la molécule plus grosse est l'hémocyanine de patelle ou l'anatoxine tétanique.


### SEQUENCE LISTING

(1) GENERAL INFORMATION:
   (i) APPLICANT: Research Corporation Technologies, Inc.
   (ii) TITLE OF INVENTION: Methods and Compositions for Delaying or Preventing the Onset of Autoimmune Disease
   (iii) NUMBER OF SEQUENCES: 4
   (iv) CORRESPONDENCE ADDRESS:
      (A) ADDRESSEE: Greenlee and Winner, P.C.
      (B) STREET: 5370 Manhattan Circle, #201
      (C) CITY: Boulder
      (D) STATE: Colorado
      (E) COUNTRY: USA
      (F) ZIP: 80303
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: WO
      (B) FILING DATE: 15-APR-1994
      (C) CLASSIFICATION:
   (vii) PRIOR APPLICATION DATA:
      (A) APPLICATION NUMBER: US 08/048,979
      (B) FILING DATE: 16-APR-1993
   (viii) ATTORNEY/AGENT INFORMATION:
      (A) NAME: Ferber, Donna M.
      (B) REGISTRATION NUMBER: 33,878
      (C) REFERENCE/DOCKET NUMBER: 17-94PCT
   (ix) TELECOMMUNICATION INFORMATION:
      (A) TELEPHONE: (303) 499-8080
      (B) TELEFAX: (303) 499-8089
      (C) TELEX: 49617824
(2) INFORMATION FOR SEQ ID NO:1:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: internal
   (xi) SEQUENCE DESCRIPTION: SEQ ID NO:1:
   Gly Ser His Leu Val Glu Ala Leu Tyr Leu Val
   1               5                   10
(2) INFORMATION FOR SEQ ID NO:2:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: N-terminal
   (xi) SEQUENCE DESCRIPTION: SEQ ID NO:2:
   Phe Val Asn Gln His Leu Cys Gly Ser
   1               5
(2) INFORMATION FOR SEQ ID NO:3:
   (i) SEQUENCE CHARACTERISTICS:
      (A) LENGTH: 30 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:3:
   Phe Val Asn Gln His Leu Cys Gly Ser His Leu Val Glu Ala Leu Tyr
   1               5                   10                  15
   Leu Val Cys Gly Glu Arg Gly Phe Phe Tyr Thr Pro Lys Thr
               20                  25                  30
(2) INFORMATION FOR SEQ ID NO:4:
   (i) SEQUENCE CHARACTERISTICS:
      (A) LENGTH: 30 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:4:
   Phe Val Asn Gln His Leu Cys Gly Ser His Leu Val Glu Ala Leu Tyr
   1               5                   10                  15
   Leu Val Cys Gly Glu Arg Gly Phe Phe Tyr Thr Pro Lys Ala
               20                  25                  30